# EUROPEAN PATENT APPLICATION

(11) **EP 3 958 200 A1**
(43) Date of publication of application: **23.02.2022**
(21) Application number: 21176338.8
(22) Date of filing: 27.05.2021
(51) Int. Cl.: G06Q 10/08, G06Q 50/22, G06Q 50/28

(54) **COMMUNICATION PROGRAM, COMMUNICATION APPARATUS AND COMMUNICATION METHOD**

(30) Priority: 21.08.2020 JP 2020139858
(71) Applicant: FUJITSU LIMITED, Kanagawa 211-8588 (JP)
(72) Inventor: NISHIMAKI, Satoru, Kawasaki-shi, 211-8588 (JP)
(74) Representative: Haseltine Lake Kempner LLP

(57) **Abstract**

A communication program included in a communication apparatus that is configured to cause a computer to execute a procedure, the procedure includes when a first process related to first data is executed, arranging, in a pointer table included in a distribution ledger by which history related to distribution of data is managed in a data distribution system which includes the communication apparatus, pointer information which associates a source identifier which is an identifier of a history item of a process related to the first data performed before the first process is executed with a next identifier which is an identifier of a history item of the first process, and synchronizing content of the pointer table with content of a pointer table of another communication apparatus.

## Description

### FIELD

The embodiments discussed herein are related to a communication program, a communication apparatus and a communication method.

### BACKGROUND

Nowadays, a mechanism for coordinating data between enterprises of various industries receives attention. For example, there is a mechanism for coordinating distribution information and medication information of medicines communicated between pharmaceutical companies, pharmaceutical wholesalers, and medical institutions which so far have been separately communicated. This coordination mechanism realizes improvement of work efficiency and appropriate division of profit based on medication results.

For data to be transmitted to or received from other organizations, traceability of the data is usually ensured to prove that the data is not maliciously processed or changed.

Techniques related to the traceability of the data are disclosed in the following.

Examples of the related art include Japanese Laid-open Patent Publication Nos. 2004-213477, 2009-123050, and 2018-160828.

To ensure data traceability, for example, there is a method by which a history item is indicated by attaching information related to processing source data to processed data. In this case, when tracing history back to a data generation source, usually, all the history items are sequentially traced. This takes time and increases processing load.

In the case where such a method is used, for example, when an enterprise that has generated the data checks how the generated data is used and processed, an inquiry is made to all enterprises in the system by broadcast communication. This takes time and exerts a large load on the network.

### SUMMARY

An object of the present disclosure is to provide a communication program, a communication method, and a communication apparatus that may efficiently perform data traceability.

According to an aspect of the embodiments, a communication program included in a communication apparatus that is configured to cause a computer to execute a procedure, the procedure includes when a first process related to first data is executed, arranging, in a pointer table included in a distribution ledger by which history related to distribution of data is managed in a data distribution system which includes the communication apparatus, pointer information which associates a source identifier which is an identifier of a history item of a process related to the first data performed before the first process is executed with a next identifier which is an identifier of a history item of the first process, and synchronizing content of the pointer table with content of a pointer table of another communication apparatus.

### BRIEF DESCRIPTION OF DRAWINGS

FIG. 1 is a diagram illustrating a configuration example of a data distribution system;
FIG. 2 is a diagram illustrating a configuration example of a GW server;
FIG. 3 is a diagram illustrating an example of a sequence of data distribution;
FIG. 4 is a diagram illustrating an example of a pointer table after a data obtaining process is completed;
FIG. 5 is a diagram illustrating an example of the pointer table after a data processing process is completed;
FIG. 6 is a diagram illustrating an example of the pointer table after a data obtaining process is completed;
FIG. 7 is a diagram illustrating an example of update timing of the pointer table;
FIG. 8 is a diagram illustrating an example of the pointer table;
FIG. 9 is a diagram illustrating an example of the pointer table;
FIG. 10 is a diagram illustrating an example of a method by which history in the past direction is able to be tracked;
FIG. 11 is a diagram illustrating an example of the pointer table that stores the source record management URL;
FIG. 12 is a diagram illustrating an example of the pointer table having undergone deletion and update; and
FIG. 13 is a diagram illustrating an example of the pointer table having undergone the deletion and update.

### DESCRIPTION OF EMBODIMENTS

Hereafter, embodiments of techniques with which data traceability may be efficiently performed will be described with reference to the drawings.

### First Embodiment

A first embodiment is described.

### [Configuration Example of Data Distribution System 1]

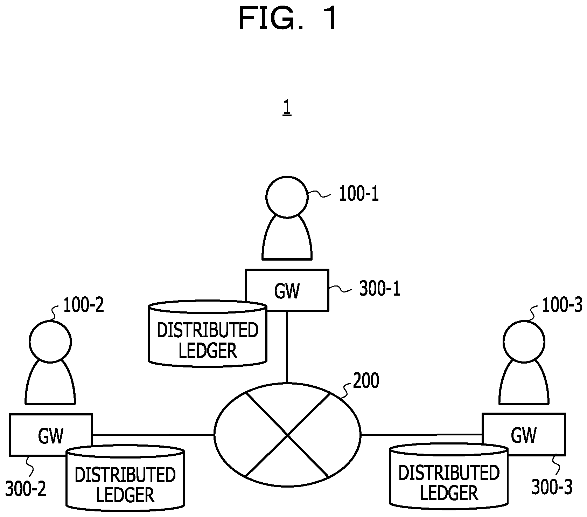

FIG. 1 is a diagram illustrating a configuration example of a data distribution system 1. The data distribution system 1 includes users 100-1 to 100-3, a network 200, and gateway (GW) servers 300-1 to 300-3. The data distribution system 1 is a communication system in which the users 100-1 to 100-3 use the GW servers 300-1 to 300-3 to distribute (transmit and receive) data. The data distribution system 1 is, for example, a communication system that uses a blockchain, and each of the GW servers 300-1 to 100-3 has a distributed ledger. The distributed ledger is a ledger that stores, for example, information such as a transaction history and disclosed data information, and the GW servers 300 share equivalent information. In the data distribution system 1, the distributed ledgers of the GW servers are synchronized with each other. For example, when the distributed ledger of a certain GW server 300 is updated, the distributed ledgers of the other GW servers 300 are also updated.

The users 100-1 to 100-3 (may be referred to as "users 100" hereafter) are users of the GW servers 300-1 to 300-3 (may be referred to as "GW servers 300" hereafter). For example, the users 100 are businesses having the GW servers 300 or administrators within the businesses. The users 100 use the GW servers 300 to communicate with the other GW servers 300 to transmit and receive data.

Each of the GW servers 300 is a communication apparatus that has a corresponding one of the distributed ledgers and, for example, a computer or a server machine. The GW servers 300 communicate with the other GW servers 300 via the network 200. The GW servers 300 synchronize the distributed ledgers via the network 200.

The network 200 is, for example, the Internet or a local network that allows data-containing packets to be transmitted and received between the GW servers 300 by using an Internet protocol (IP) address or the like.

In the data distribution system 1, when one of the GW servers 300 generates the data, the GW server 300 register the generated data in the distributed ledger and discloses the generated data to the other GW servers 300. When the GW servers 300 recognize the disclosed data, for example, the GW servers 300 perform transaction with the GW server 300 that is a generation source of the data and obtains the data.

### [Configuration Example of GW Server 300]

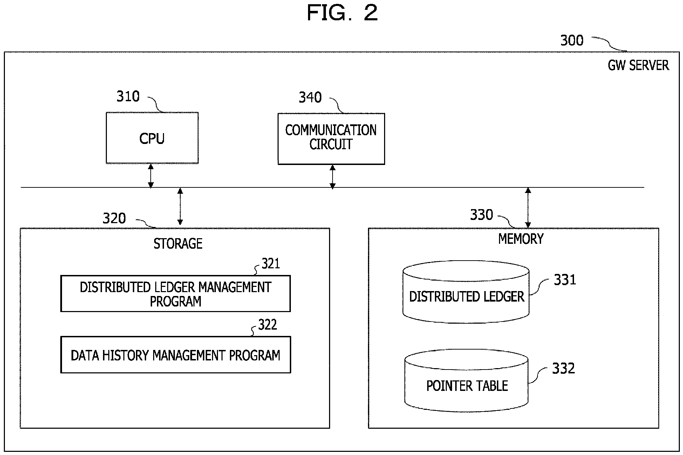

FIG. 2 is a diagram illustrating a configuration example of each of the GW servers 300. The GW server 300 includes a central processing unit (CPU) 310, a storage 320, a memory 330, and a communication circuit 340.

The storage 320 is an auxiliary storage device such as a flash memory, a hard disk drive (HDD), or a solid-state drive (SSD) that stores programs and data. The storage 320 stores a distributed ledger management program 321 and a data history management program 322.

The memory 330 is an area into which the programs stored in the storage 320 are loaded. The memory 330 may also be used as an area in which the programs store data. The memory 330 stores a distributed ledger 331 and a pointer table 332.

The distributed ledger 331 is a ledger that stores information related to the disclosed data and the transaction history of the data. The distributed ledgers 331 are included in the respective GW servers 300, are synchronized with each other, and store equivalent content.

The pointer table 332 is a table that stores pointer information including, for example, a data processing history item and a data transaction history item. For example, the pointer table 332 stores information regarding states of the data in data processing before and after the processing and a transmission source and a transmission destination in data transaction. Details of the pointer table 332 will be described later.

The CPU 310 is a processor that loads the programs stored in the storage 320 into the memory 330 and executes the loaded programs to construct units and realize processes.

The communication circuit 340 is a circuit that communicates with the other GW servers 300 via the network 200. The communication circuit 340 is, for example, a network interface card (NIC). The communication circuit 340 may be coupled to the network 200 in a wireless manner or a wired manner.

The CPU 310 executes the distributed ledger management program 321 to construct a synchronization unit and perform a distributed ledger management process. The distributed ledger management process is a process of updating the distributed ledger or synchronizing the distributed ledgers between the GW servers 300.

The CPU 310 executes the data history management program 322 to construct a storage unit and the synchronization unit and perform a data history management process. The data history management process is a process of managing the pointer table 332. In the data history management process, the GW server 300 updates the pointer table 332 and synchronizes the pointer table 332 with the pointer tables 332 included in the other GW servers 300. The synchronization of the pointer tables 332 is realized by, for example, a similar method to a method for synchronization of the distributed ledgers. For example, when the pointer table 332 is included in part of the distributed ledger, the synchronization of the distributed ledgers simultaneously synchronizes the pointer tables 332.

### [About Data History Management Process]

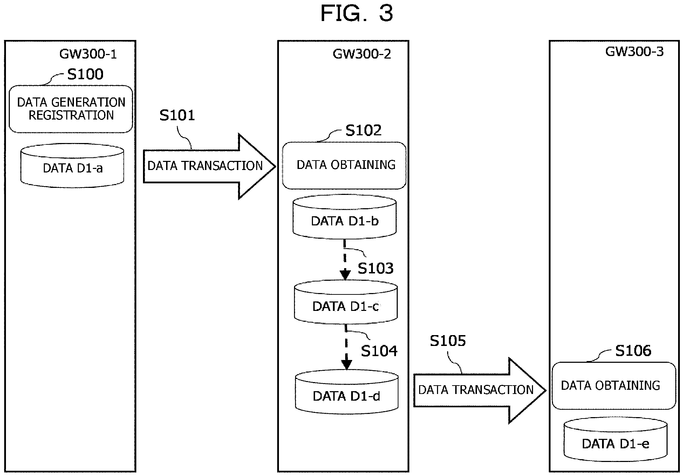

The data history management process is described. FIG. 3 is a diagram illustrating an example of a sequence of the data distribution. FIG. 3 is the example of the sequence in which data D1 generated by the GW server 300-1 is delivered to the GW server 300-2, updated (processed) by the GW server 300-2, and further, delivered to the GW server 300-3. "Data D1-x" illustrated in FIG. 3 indicates, for example, the states of the data before and after a certain process. The "data D1-x" may be understood as an identifier of a history item (record) indicating that a certain process has been executed on the data D1. Assignment of x is performed in a chronological sequence in an alphabetical order from "a". In the embodiments hereafter, unless otherwise specified, description is made for a case, as an example, where processes in the sequence illustrated in FIG. 3 are executed.

The GW server 300-1 generates data and executes data generation registration to be registered (S100). The GW server 300-1 generates and registers data D1. This generated data D1 is referred to as data D1-a. When the data D1-a is registered, it is recognized that the other GW servers 300 are generated in the GW server 300-1, and the other GW servers 300 become able to be obtained.

The GW server 300-2 recognizes the generation of the data D1 and attempts to obtain the data D1. The GW server 300-2 performs, for example, a transaction with the GW 300-1 (S101) to obtain the data. For example, in the transaction, the GW server 300-2 transmits a request for obtaining of the disclosed data D1 by the GW server 300-2 to the GW server 300-1. The GW server 300-1 accepts (or rejects) the request of the GW server 300-2. The GW server 300-2 obtains the data D1 in a transaction (S102) and internally manages the data as data D1-b.

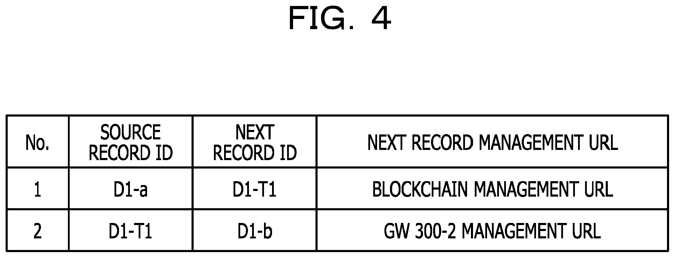

FIG. 4 is a diagram illustrating an example of the pointer table 332 after the data obtaining process S102 is completed. The state of the pointer table 332 is described with reference to FIG. 4. The pointer table 332 is a table that stores pointer information in which history items (records) before and after a certain process are associated with each other. The pointer table 332 stores a number ("No."), a source record identification ("SOURCE RECORD ID"), a next record identification ("NEXT RECORD ID"), and a next record management Uniform Resource Locator ("NEXT RECORD MANAGEMENT URL").

The "No." indicates pointer numbers. The pointer numbers are, for example, sequentially assigned from 1 in the order of occurrences.

The "SOURCE RECORD ID" is an identifier (source identifier) of a record of a process executed before (immediately before, preceding) a certain process (transaction, processing, obtaining, or the like). The "NEXT RECORD ID" is an identifier (next identifier) of a record of a certain process (transaction, processing, obtaining, or the like). The record is a history item of data delivery or data processing such as a transaction, obtaining, or processing of the data. For example, the "SOURCE RECORD ID" is an identifier of a record of a transaction, obtaining, or processing executed before a certain reference process, and the "NEXT RECORD ID" is an identifier of a record of a certain reference process.

The "NEXT RECORD MANAGEMENT URL" indicates a URL that manages the next record, and, by using the URL, the history item of the next record is able to be checked.

A pointer number 1 associates "D1-a" as the "SOURCE RECORD ID" indicating a record of generation of the data D1 with "D1-T1" as the "NEXT RECORD ID" indicating a record of a transaction of the data D1. For example, the pointer number 1 indicates that the generated data D1-a is transacted in a transaction T1. The pointer number 1 stores, as the "NEXT RECORD MANAGEMENT URL", a management URL of a blockchain (or the data distribution system 1, the distributed ledger) that manages the transaction history. The pointer number 1 is stored in the pointer table 332 by the GW server 300-1 when, for example, the data transaction S101 is established between the GW server 300-1 and the GW server 300-2.

A pointer number 2 associates "D1-T1" as the "SOURCE RECORD ID" indicating a record of a transaction of the data D1 with "D1-b" as the "NEXT RECORD ID" indicating a record of obtaining of the data D1. For example, the pointer number 2 indicates that the data D1 transacted in the transaction T1 is obtained and stored as the data D1-b. The pointer number 2 stores the management URL of the GW server 300-2 that manages the data D1-b as the "NEXT RECORD MANAGEMENT URL". The pointer number 2 is stored in the pointer table 332 by the GW server 300-2 when, for example, the GW server 300-2 executes the obtaining process S102 of the data D1 accompanying the data transaction S101.

The data transaction is a contract to transmit and receive the data conducted between the user 100 of one of the GW server 300 that is to obtain the data and the user 100 of the other GW server 300 that is to deliver the data. For example, communication in which the user 100 who desires to obtain certain disclosed data requests for obtaining to the user who discloses the certain data and the user 100 who discloses the certain data admits (or does not admit) the obtaining is performed.

Referring back to the sequence illustrated in FIG. 3, the GW server 300-2 processes the data D1-b (S103) and generates data D1-c. The GW server 300-2 further processes the data D1-c (S104) and generates data D1-d.

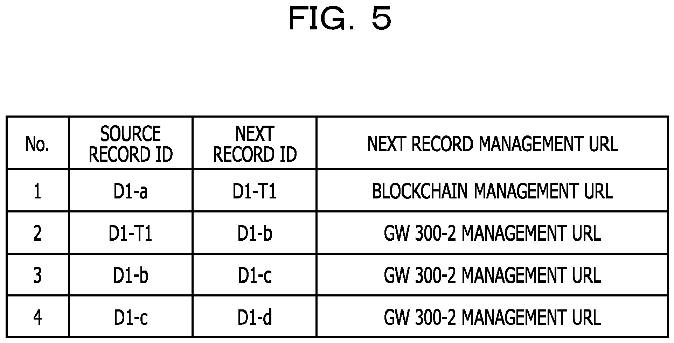

FIG. 5 is a diagram illustrating an example of the pointer table 332 after the data processing process S104 is completed. A pointer number 3 associates "D1-b" as the "SOURCE RECORD ID" indicating a record from which the data D1 has been obtained with "D1-c" as the "NEXT RECORD ID" indicating a record into which the data D1 has been processed. For example, the pointer number 3 indicates that the obtained data D1-b is processed and managed as D1-c. In the pointer number 3, a management URL of the GW server 300-2 that manages the processed data D1 is stored as the "NEXT RECORD MANAGEMENT URL". For example, the pointer number 3 is added to the pointer table 332 by the GW server 300-2 when the GW server 300-2 processes the data D1 in the processing process S103.

A pointer number 4 associates "D1-c" as the "SOURCE RECORD ID" indicating a record into which the data D1 has been processed with "D1-d" as the "NEXT RECORD ID" indicating a record into which the data D1 has been processed. For example, the pointer number 4 indicates that the processed data D1-c is further processed and managed as D1-d. In the pointer number 4, the management URL of the GW server 300-2 that manages the processed data D1 is stored as the "NEXT RECORD MANAGEMENT URL". For example, the pointer number 4 is added to the pointer table 332 by the GW server 300-2 when the GW server 300-2 processes the data D1 in the processing process S104.

Referring back to the sequence illustrated in FIG. 3, the GW server 300-3 attempts to obtain the data D1-d managed by the GW server 300-2. The GW server 300-3 performs, for example, a transaction to obtain the data with the GW 300-2 (S105). The GW server 300-3 obtains the data D1 in the transaction (S106) and internally manages the data D1 as data D1-e.

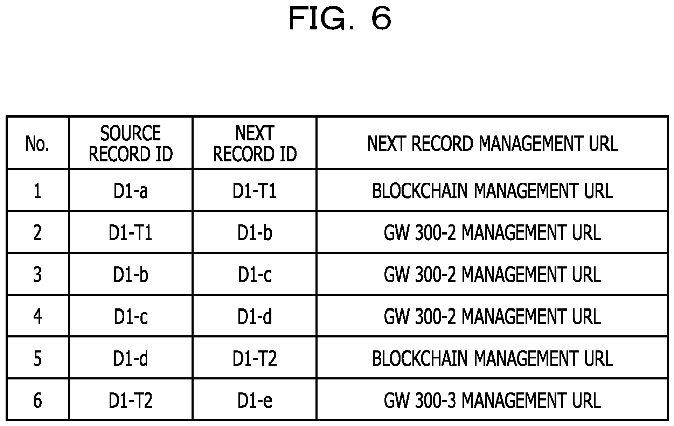

FIG. 6 is a diagram illustrating an example of the pointer table 332 after the data obtaining process S106 is completed. A pointer number 5 associates the "D1-d" as the "SOURCE RECORD ID" indicating a record into which the data D1 is processed with the "D1-T2" as the "NEXT RECORD ID" indicating a record with which the data D1 is transacted. For example, the pointer number 5 indicates that the processed data D1-d is transacted in a transaction T2. The pointer number 5 stores, as the "NEXT RECORD MANAGEMENT URL", the management URL of the blockchain that manages the transaction history. The pointer number 5 is stored, for example, in the pointer table 332 by the GW server 300-2 when the data transaction S105 is established between the GW server 300-2 and the GW server 300-3.

A pointer number 6 associates the "D1-T2" as the "SOURCE RECORD ID" indicating a record in which the data D1 is transacted with the "D1-e" as the "NEXT RECORD ID" indicating a record in which the data D1 is obtained. For example, the pointer number 6 indicates that the data D1 transacted in the transaction T2 is obtained and stored as the data D1-e. The pointer number 6 stores, as the "NEXT RECORD MANAGEMENT URL", a management URL of the GW server 300-3 that manages the data D1-e. The pointer number 6 is stored, for example, in the pointer table 332 by the GW server 300-3 when the GW server 300-3 executes the obtaining process S106 of the data D1 accompanying the data transaction S105.

According to the first embodiment, since the GW server 300 includes the pointer table 332, history of the data is able to be tracked in the time direction. For example, when tracking how the generated data 1 has been changed, the GW server 300-1 detects the pointer number 2 the source record ID of which is D1-T1 which is the next record ID of the pointer number 1. From the records of the pointer number 2, the GW server 300-1 is able to recognize that the data D1-a is transacted in the transaction T1 and is managed (has been managed) as the data D1-b by the GW server 300-2. The GW server 300-1 similarly follows the records of the pointer table 332 and finally detects the pointer number 6. Thus, the GW server 300-1 is able to recognize that the data D1-a is managed as the data D1-e by the GW server 300-3. When the GW server 300-1 desires to check the details of the data D1-e, the GW server 300-1 is able to check with the management URL of the GW server 300 of the next record management URL of the pointer number 6.

### Second Embodiment

Next, a second embodiment is described. The GW servers 300 according to the second embodiment updates the pointer table 332 at different timing from that of the first embodiment.

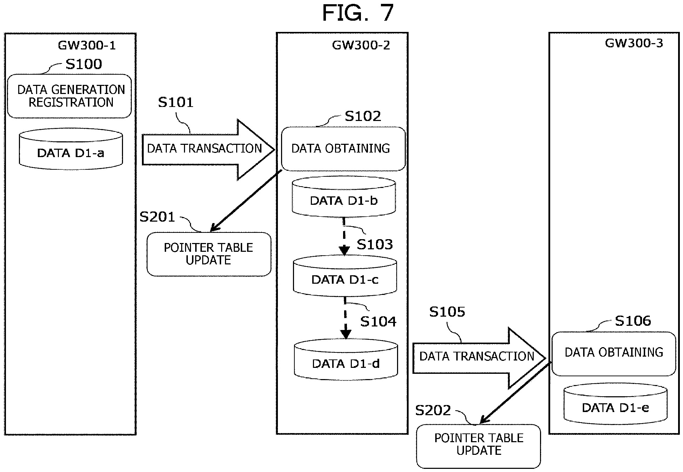

FIG. 7 is a diagram illustrating an example of update timing of the pointer table. The GW servers 300 update the pointer table 332 when the data is moved to the other GW servers 300. The GW servers 300 update the pointer table 332 when delivering the data and when obtaining the data.

The pointer information of the pointer numbers 1 and 2 is updated (S201) when the data obtaining process S102 is executed. The GW server 300-1 adds the pointer information of the pointer number 1 to the pointer table 332 when the data D1 is delivered in the data obtaining process S102. The GW server 300-2 adds the pointer information of the pointer number 2 to the pointer table 332 when the data D1 is obtained in the data obtaining process S102.

The pointer information of the pointer numbers 3 to 6 is updated (S202) when the data obtaining process S106 is executed. The GW server 300-2 adds the pointer information of the pointer numbers 3 to 5 to the pointer table 332 when the data D1 is delivered in the data obtaining process S106. The GW server 300-3 adds the pointer information of the pointer number 6 to the pointer table 332 when the data D1 is obtained in the data obtaining process S106.

According to the second embodiment, updating of the pointer table 332 is triggered by the movement of the data. Thus, the number of times of updating the pointer table may be reduced, the number of times of communication for synchronizing the pointer tables 332 may be suppressed, and the load on the network may be reduced. According to the second embodiment, for example, the timing of registration of the history items of the processing processes S103 and S104 in the pointer table 332 is delayed compared to that of the first embodiment. However, since the history items of the processing processes are able to be obtained from the management URL of the GW server 300-2 that performs the processing processes, the other GW servers 300 are able to track the latest history.

### Third Embodiment

Next, a third embodiment is described. The pointer table 332 according to the third embodiment stores different pointer information. The pointer table 332 does not store the history item of the processing process in the GW server 300.

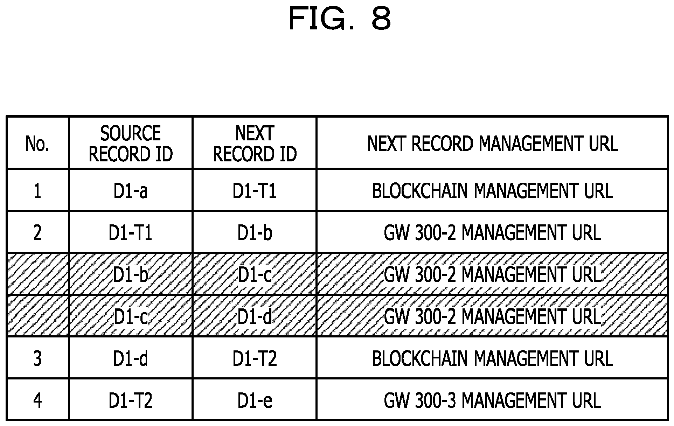

FIG. 8 is a diagram illustrating an example of the pointer table 332. According to the third embodiment, the GW servers 300 do not store the pointer information in a shaded area of the pointer table 332 illustrated in FIG. 8. Thus, the storage amount of the pointer table 332 may be reduced.

The next record ID of the pointer information of the pointer number 2 is "D1-b", and the source record ID of the pointer information of the pointer number 3 is "D1-d". Thus, the pointer information of the pointer numbers 2 and 3 are not linked. However, for example, the GW server 300-1 is able to obtain the management URL of the GW server 300-2 from the pointer information of the pointer number 2. By using the management URL of the GW server 300-2, the GW server 300-1 is able to obtain an internal history item of the GW server 300-2, for example, the GW server 300-1 is able to obtain the fact that the GW server 300-2 performs processing therein and the data after the processing is managed as D1-d (record D1-d). Since the GW server 300-1 is able to obtain the pointer information of the pointer number 3 from the record D1-d, it is possible to track the data D1 thereafter.

### Fourth Embodiment

Next, a fourth embodiment is described. The pointer table 332 according to the fourth embodiment stores different pointer information. When data processing is performed a plurality of times in the GW server 300, the pointer table 332 stores only a final (last processing before a transaction) history item.

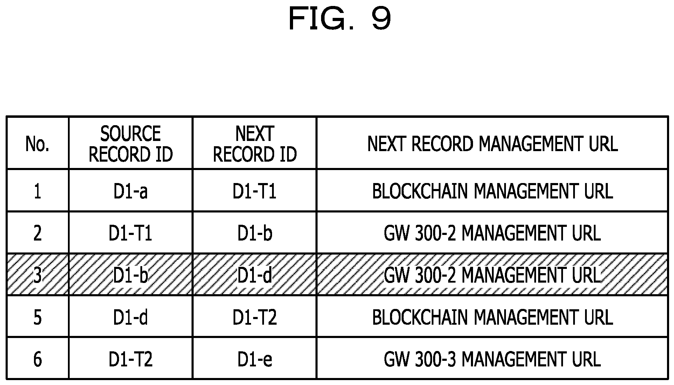

FIG. 9 is a diagram illustrating an example of the pointer table 332. According to the fourth embodiment, the GW server 300 stores the history item of final processing as pointer information. This may reduce the storage amount of the pointer table 332 when the processing is performed a plurality of times in the GW server 300.

The GW server 300-2 does not store the pointer information in the pointer table 332 when the processing processes S103 and S104 are performed. For example, when the data transaction S105 is performed, the GW server 300-2 stores the processing processes S103 and S104 as pointer information of a single occurrence in the pointer number 3. The pointer information of the pointer number 3 stores the record "D1-b" of the obtained data as the source record ID and the record "D1-d" of the data after the two processing processes S103 and S104 as the next record ID. The management URL of the GW server 300-2 is stored as the next record management URL.

According to the fourth embodiment, the pointer table 332 stores part of the processing history. According to the third embodiment, the pointer information of the pointer table 332 is not necessarily linked, and the GW server 300 may obtain internal history from the other GW server 300 by using the management URL.

However, according to the fourth embodiment, the amount of information stored in the pointer table 332 is reduced, loss of linkage in the pointer information is avoided, and the GW server 300 is able to track the history without using the management URL.

### Fifth Embodiment

Next, a fifth embodiment is described. In the above-described embodiments, the case where the GW server 300 tracks the history (history in the future direction) indicating how the data has been changed overtime has been described. However, in some cases, the GW server 300 tracks the history (history in the past direction) indicating what kind of processes has been performed on certain data to obtain the data.

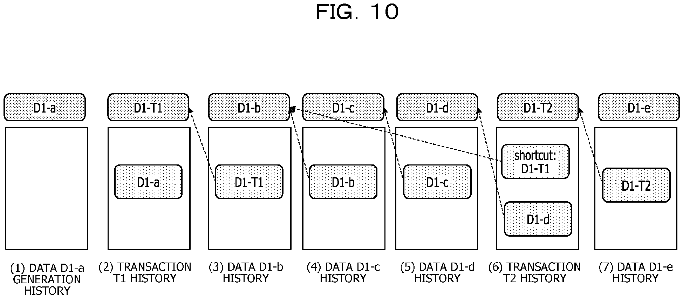

FIG. 10 is a diagram illustrating an example of a method by which the history in the past direction is able to be tracked. For example, the GW server 300 assigns the history information to the data D1. Shaded quadrangles in lower parts indicate history information, and upper quadrangles indicate identifiers of the history information (or an identifiers of data, a transaction, a process, or the like).

The GW server 300-1 creates the history information when the data D1 is generated and assigns this history information to the data D1. The identifier of the assigned history information is "D1-a" ((1) in FIG. 10).

The GW server 300-1 performs the transaction T1 (transaction process S101) and creates new history information. In the history information, "D1-a" being the identifier of the previous history information is stored. The identifier of the assigned history information is "D1-T1" ((2) in FIG. 10).

The GW server 300-2 obtains the data D1 in the transaction T1 (obtaining process S102). At this time, the GW server 300-2 creates new history information. In the history information, "D1-T1" being the identifier of the previous history information is stored. The identifier of the assigned history information is "D1-b" ((3) in FIG. 10).

Thereafter, in each process, the GW server 300 creates history information and attaches the identifier of the history information. Since the identifiers of the previous history information are stored in the history information, the GW server 300 is able to track the past history by tracing back the identifiers of the history information.

FIG. 10 illustrates "shortcut: D1-T1" in (6). This indicates shortcut information. The shortcut information includes, for example, the identifier of history information in the previous transaction. When only the movement history item of the data is obtained, by tracking the shortcut information, the GW server 300 is able to obtain how the data is moved.

When the method illustrated in FIG. 10 and the processes according to the first to fourth embodiments are combined, the GW server 300 is able to track the history in the past direction and in the future direction.

However, in the method illustrated in FIG. 10, an inquiry using the management URL of each GW server 300 or the blockchain is used every time the history information is traced back. Accordingly, the management URL of the source record ID is stored in the pointer table 332 so that the history in the past direction is able to be tracked as is the case with the tracing of the history in the future direction.

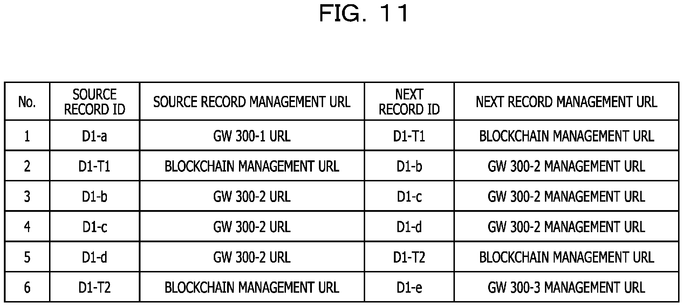

FIG. 11 is a diagram illustrating an example of the pointer table 332 that stores the source record management URL. The GW server 300 stores "SOURCE RECORD MANAGEMENT URL" as the pointer information. The "SOURCE RECORD MANAGEMENT URL" indicates a URL that manages the source record, and the history item of the source record is able to be checked by using the URL.

For example, the source record management URL of the pointer information of the pointer number 2 is the management URL of the blockchain that manages "D1-T1". Regarding the pointer information of other pointer numbers, the source record management URLs are similarly stored as the pointer information.

According to the fifth embodiment, the GW server 300 is able to track the history also in the past direction by using the pointer table 332. For example, the GW server 300-3 checks the pointer table 332 for checking what kind of processes has been performed on the data D1-e. For example, the GW server 300-3 is able to recognize, from the pointer information of the pointer number 5, that the data D1-d before the transaction T2 is performed is managed by the GW server 300-2 of the source record management URL. The GW server 300-3 is able to obtain the history item related to the data D1-d from the GW server 300-2 by using the source record management URL.

### Sixth Embodiment

Next, a sixth embodiment is described. According to the sixth embodiment, the GW server 300 performs a deletion process that deletes predetermined pointer information from the pointer table 332. This may suppress the amount of data of the pointer table 332.

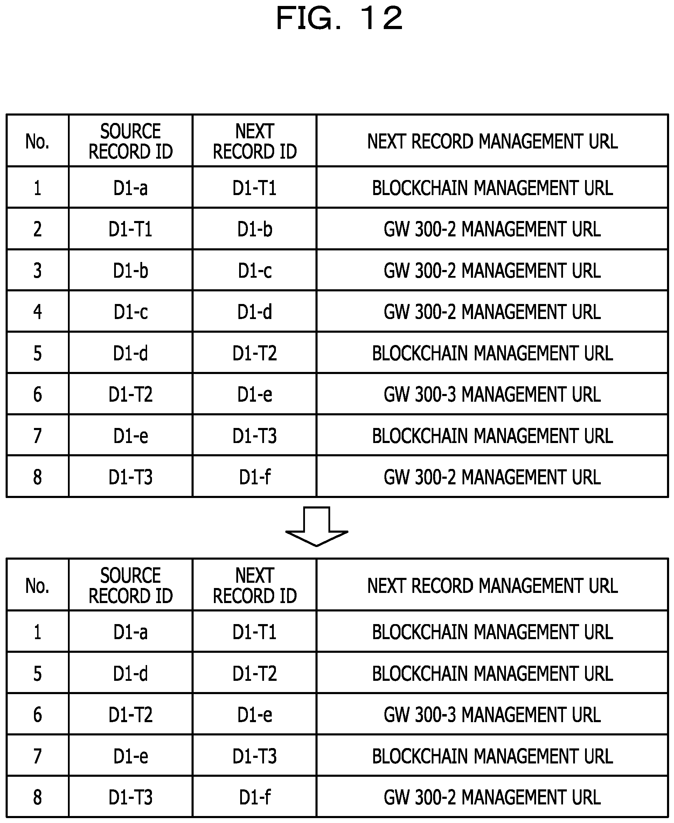

FIG. 12 is a diagram illustrating an example of the pointer table 332 having undergone deletion and update. An upper portion of FIG. 12 illustrates a state before the deletion and update are performed. A lower portion of FIG. 12 illustrates a state after the deletion and update have been performed. When a plurality of management URLs of the same GW server 300 exist as the next record management URL in the pointer table 332, the GW server 300 retains only the latest pointer information (the pointer number is the largest) in the pointer table 332 and deletes the other pointer information having the management URL of the same GW server 300. In the upper portion of FIG. 12, the management URL of the GW server 300-2 exists at the pointer numbers 2, 3, 4, 8. In this case, the GW server 300 retains only the pointer information of the pointer number 8 and deletes the pointer information of the other pointer numbers 2, 3, 4. After the deletion and update, the pointer table 332 is in the state illustrated in the lower portion of FIG. 12.

The GW server 300 does not delete the blockchain management URL even when there are a plurality of blockchain management URLs. For example, since the blockchain manages data registration and transactions of all the GW servers 300 and performs processes such as synchronization of ledgers, the processing load is large. Accordingly, for suppressing the number of times of inquiries to the blockchain as much as possible, the pointer information of the blockchain is retained in the pointer table 332.

The GW server 300 is able to track the history in the future direction by using the pointer table 332 after the deletion and update. For example, when the next record ID is not linked to the source record ID (a certain next record ID does not exist in the source record ID of another piece of the pointer information), history thereafter is able to be tracked by using the next record management URL. When the deletion and update are not performed, all the pointer information is retained. In this case, the GW server 300 is able to track up to the latest history item without using the management URL. In contrast, when the deletion and update are performed, in some cases, the GW server 300 is unable to track up to the latest history item without an inquiry made by using the management URL. However, the amount of data of the pointer table 332 may be reduced.

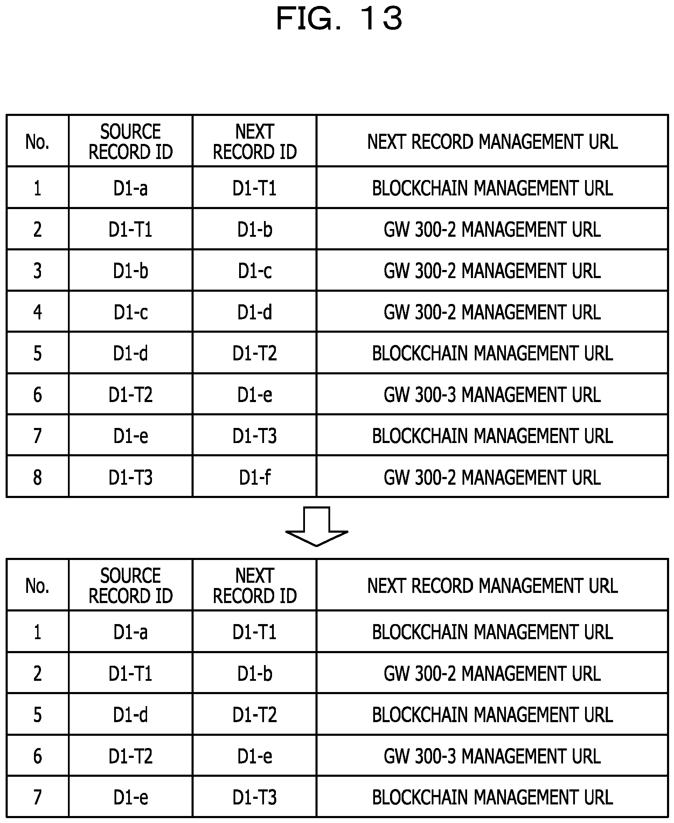

FIG. 13 is a diagram illustrating an example of the pointer table 332 having undergone the deletion and update. An upper portion of FIG. 13 illustrates a state before the deletion and update are performed. A lower portion of FIG. 13 illustrates a state after the deletion and update have been performed. When a plurality of management URLs of the same GW server 300 exist as the next record management URL in the pointer table 332, the GW server 300 retains only the oldest pointer information (the pointer number is the smallest) in the pointer table 332 and deletes the other pointer information having the management URL of the same GW server 300. In the upper portion of FIG. 13, the management URL of the GW server 300-2 exists at the pointer numbers 2, 3, 4, 8. In this case, the GW server 300 retains only the pointer information of the pointer number 2 and deletes the pointer information of the other pointer numbers 3, 4, 8. After the deletion and update, the pointer table 332 is in the state illustrated in the lower portion of FIG. 13.

### [Modification Example]

When the pointer table 332 is updated, the synchronization process is performed. Accordingly, communication occurs in the blockchain. In the synchronization process, as the number of GW servers 300 increases, the amount of communication increases, and a communication load of the network increases. For example, when the above-described deletion and update process is executed, the communication load increases in some cases.

Thus, according to the modification example, the GW server 300 performs an extraction process by which the pointer information is extracted instead of performing the deletion and update on the pointer table 332. The GW server 300 performs the extraction process to extract the pointer information illustrated in the lower portion of FIG. 12 or the lower portion of FIG. 13. The GW server 300 tracks the history by using the extracted pointer information. In this way, the GW server 300 is able to track the history based on the selected pointer information without deleting and updating the pointer table 332. For example, the GW server 300 may store the extracted pointer information therein for a predetermined period of time. The GW server 300 may periodically extract pointer information related to related data from the pointer table 332.

## Claims

1. A communication program included in a communication apparatus that is configured to cause a computer to execute a procedure, the procedure comprising:
when a first process related to first data is executed, arranging, in a pointer table included in a distribution ledger by which history related to distribution of data is managed in a data distribution system which includes the communication apparatus, pointer information which associates a source identifier which is an identifier of a history item of a process related to the first data performed before the first process is executed with a next identifier which is an identifier of a history item of the first process; and
synchronizing content of the pointer table with content of a pointer table of another communication apparatus.

2. The communication program according to claim 1, wherein the pointer information includes a management Uniform Resource Locator (URL) of a history item of execution of the first process.

3. The communication program according to claim 1, the process further comprising:
detecting, from the pointer table, the pointer information which includes, as the source identifier, an identifier of a history item of a second process related to second data;
acquiring the next identifier of the detected pointer information; and
tracking the history item of the second process.

4. The communication program according to any of claims 1 to 3, wherein the first process includes:
a transaction process configured to request and accept an acquisition of the first data, and
an acquisition process configured to acquire the first data in accordance with the transaction process.

5. The communication program according to claim 4, wherein the first process further includes a processing process configured to process the first data.

6. The communication program according to claim 5,
wherein, when the transaction process or the acquisition process is executed,
the procedure arranges, in the pointer table, :
pointer information which includes identifiers related to the transaction process and the acquisition process, and
pointer information which includes an identifier related to the processing process.

7. The communication program according to claim 5,
wherein, when the processing process is executed a plurality of times in the communication apparatus itself after the acquisition process has been executed,
the procedure arranges, in the pointer table, the pointer information which associates an identifier of a history item of the acquisition process set as the source identifier with an identifier of a history item of the processing process executed at last out of the executed plurality of times of the processing process set as the next identifier, and
an identifier of a history item of the processing process other than the processing process executed at last out of the executed plurality of times of the processing process is not arranged in the pointer table.

8. The communication program according to any of the preceding claims, wherein the procedure synchronizes the content of the distributed ledger with content of a distributed ledger of the another communication apparatus.

9. The communication program according to claim 2, wherein the pointer information further includes a management URL of a history item of execution of a process which corresponds to the source identifier.

10. The communication program according to claim 2, the procedure further comprising:
deleting, from the pointer table, when an identical management URL exists in a plurality of pieces of the pointer information, a piece of the pointer information other than a piece of the pointer information arranged at last in the pointer table.

11. The communication program according to claim 2, the procedure further comprising:
deleting, from the pointer table, when an identical management URL exists in a plurality of pieces of the pointer information, a piece of the pointer information other than a piece of the pointer information arranged at first in the pointer table.

12. The communication program according to claim 2, the procedure further comprising:
extracting, from the pointer table, when an identical management URL exists in a plurality of pieces of the pointer information, a single piece of the pointer information, and
tracking a history item of a process related to a third data by using the extracted piece of the pointer information.

13. A communication apparatus comprising:
a storage unit configured to, when a first process related to first data is executed, arrange, in a pointer table included in a distribution ledger by which history related to distribution of data is managed in a data distribution system which includes the communication apparatus, pointer information which associates a source identifier which is an identifier of a history item of a process related to the first data performed before the first process is executed with a next identifier which is an identifier of a history item of the first process; and
a synchronization unit configured to synchronize content of the pointer table with content of a pointer table of another communication apparatus.

14. A communication method comprising:
when a first process related to first data is executed, arranging, in a pointer table included in a distribution ledger by which history related to distribution of data is managed in a data distribution system which includes the communication apparatus, pointer information which associates a source identifier which is an identifier of a history item of a process related to the first data performed before the first process is executed with a next identifier which is an identifier of a history item of the first process; and
synchronizing content of the pointer table with content of a pointer table of another communication apparatus, by a processor.

## Amended claims

### Amended claims in accordance with Rule 137(2) EPC.

1. A communication program included in a communication apparatus (300) that is configured to cause a computer to execute a procedure, the procedure comprising:
when a first process related to first data is executed, arranging, in a pointer table (332) included in a distribution ledger by which history related to distribution of data is managed in a data distribution system (1) which includes the communication apparatus (300), pointer information which associates a source identifier which is an identifier of a history item of a process related to the first data performed before the first process is executed with a next identifier which is an identifier of a history item of the first process; and
synchronizing content of the pointer table (332) with content of a pointer table of another communication apparatus (300-2),
wherein the process related to the first data performed before the first process includes:
a transaction process configured to request and accept an acquisition of the first data, and
an acquisition process configured to acquire the first data in accordance with the transaction process,
wherein the first process includes data processing of the first data,
wherein, when data processing of the first data is executed a plurality of times in the communication apparatus (300) itself after the acquisition process has been executed, the procedure arranges, in the pointer table (332), the pointer information which associates
an identifier, set as the source identifier, of a history item of the acquisition process with
an identifier, set as the next identifier, of a history item of the data processing of the first data which was executed last out of the plurality of times data processing of the first data was executed,
wherein an identifier of a history item of data processing of the first data other than the data processing of the first data which was executed last out of the plurality of times data processing of the first data was executed is not arranged in the pointer table (332).

2. The communication program according to claim 1, wherein the pointer information includes a management Uniform Resource Locator (URL) of a history item of execution of the first process.

3. The communication program according to claim 1, the process further comprising:
detecting, from the pointer table (332), the pointer information which includes, as the source identifier, an identifier of a history item of a second process related to second data;
acquiring the next identifier of the detected pointer information; and
tracking the history item of the second process.

4. The communication program according to claim 1, wherein the procedure synchronizes the content of the distributed ledger with content of a distributed ledger of the another communication apparatus (300-2).

5. The communication program according to claim 2, wherein the pointer information further includes a management URL of a history item of execution of a process which corresponds to the source identifier.

6. A communication apparatus (300) comprising:
a storage unit (310) configured to, when a first process related to first data is executed, arrange, in a pointer table (332) included in a distribution ledger by which history related to distribution of data is managed in a data distribution system (1) which includes the communication apparatus (300), pointer information which associates a source identifier which is an identifier of a history item of a process related to the first data performed before the first process is executed with a next identifier which is an identifier of a history item of the first process; and
a synchronization unit (310) configured to synchronize content of the pointer table (332) with content of a pointer table of another communication apparatus (300-2),
wherein the process related to the first data performed before the first process includes:
a transaction process configured to request and accept an acquisition of the first data, and
an acquisition process configured to acquire the first data in accordance with the transaction process,
wherein the first process includes data processing of the first data,
wherein the storage unit (310) is configured to arrange, when data processing of the first data is executed a plurality of times in the communication apparatus (300) itself after the acquisition process has been executed, in the pointer table (332), the pointer information which associates
an identifier, set as the source identifier, of a history item of the acquisition process with
an identifier, set as the next identifier, of a history item of the data processing of the first data which was executed last out of the plurality of times data processing of the first data was executed,
wherein an identifier of a history item of data processing of the first data other than the data processing of the first data which was executed last out of the plurality of times data processing of the first data was executed is not arranged in the pointer table (332).

7. A communication method comprising:
when a first process related to first data is executed,
arranging, in a pointer table (332) included in a distribution ledger by which history related to distribution of data is managed in a data distribution system (1) which includes the communication apparatus (300), pointer information which associates a source identifier which is an identifier of a history item of a process related to the first data performed before the first process is executed with a next identifier which is an identifier of a history item of the first process; and
synchronizing content of the pointer table (332) with content of a pointer table of another communication apparatus (300-2), by a processor,
wherein the process related to the first data performed before the first process includes:
a transaction process configured to request and accept an acquisition of the first data, and
an acquisition process configured to acquire the first data in accordance with the transaction process,
wherein the first process includes data processing of the first data,
wherein the communication method comprises, when data processing of the first data is executed a plurality of times in the communication apparatus (300) itself after the acquisition process has been executed, arranging, in the pointer table (332), the pointer information which associates
an identifier, set as the source identifier, of a history item of the acquisition process with
an identifier, set as the next identifier, of a history item of the data processing of the first data which was executed last out of the plurality of times data processing of the first data was executed,
wherein an identifier of a history item of data processing of the first data other than the data processing of the first data which was executed last out of the plurality of times data processing of the first data was executed is not arranged in the pointer table (332).
